# EUROPÄISCHE PATENTSCHRIFT

(11) **EP 0 582 857 B1**
(45) Veröffentlichungstag und Bekanntmachung des Hinweises auf die Patenterteilung: **05.02.1997**
(21) Anmeldenummer: 93111522.4
(22) Anmeldetag: 19.07.1993
(51) Int. Cl.: A61B 17/58

(54) **Wirbelsäulenfixationselement**
Spinal fixation element
Elément de fixation du rachis

(30) Priorität: 12.08.1992 CH 522/92
(43) Veröffentlichungstag der Anmeldung: 16.02.1994
(73) Patentinhaber: Synthes AG, Chur, CH-7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- DE-A- 4 021 246
- DE-A- 4 102 462
- FR-A- 2 309 198
- US-A- 4 805 602

## Beschreibung

Die Erfindung bezieht sich auf ein Wirbelsäulenfixationselement, gemäss der Gattung des Patentanspruchs 1.

Ein Wirbelsäulenfixationselement dieser Gattung ist beispielsweise in der US-A-4805602 beschrieben.

Für die posteriore Wirbelsäulen-Korrektur gibt es grundsätzlich zwei Arten von Verankerungselementen. Einerseits die Pedikelschrauben, andererseits die Wirbelsäulenhaken. Die Pedikelschrauben werden, wie es schon ihr Name sagt, in die Pedikel der Wirbelsäule eingebracht. Die Haken können entweder an die Pedikel oder an die Lamina der Wirbelkörper angebracht werden. Sie gelangen vor allem im Bereich der thorakalen Wirbelsäule zum Einsatz. Bei der anterioren Wirbelsäulen-Korrektur werden vor allem Schrauben verwendet, die in den Wirbelkörper eingesetzt werden.

Das Problem bei allen bisher bekannten Wirbelsäulensysteme, zur Behandlung von Wirbelsäulen-Deformationen, ist ihre Kompliziertheit in der Anwendung. Dieses Problem zeigt sich vor allem, wenn die einzelnen Wirbelsäulen-Verankerungen mit einem Längsträger verbunden werden müssen. Entgegen der Behandlung von Frakturen der Wirbelsäule, müssen bei der Behandlung von Deformationen mehrere der eingangs definierten Verankerungselemente an der Wirbelsäule fixiert werden. Da die Wirbelsäule deformiert ist, ist es in den seltensten Fälle möglich, diese Verankerungselemente in einer Linie zu setzen. Daraus ergibt sich das Problem der Adaptation mit einem Längsträger, der nach dem Stand der Technik aus einem Rundstab besteht.

Bei der sogenannten Derotationstechnik wird der Längsträger vorgebogen und nach erfolgter Fixation an die Verankerungselemente, um 90 Grad gedreht. Daraus erfolgt aus einer seitlichen Krümmung der Wirbelsäule eine wiederum anatomische Krümmung in der sagittalen Ebene. Trotz der anfänglichen Begeisterung für diese Technik, musste festgestellt werden, dass die Langzeitresultate nur eine geringe Korrektur aufzeigten.

Eine, nach dem heutigen Stand der Technik optimalere Korrektur der Wirbelsäule kann mit der sogenannten Rahmentechnik erreicht werden. Bei dieser Technik wird der zu korrigierende Wirbelsäulenteil durch zwei Längsträger überbrückt. Die beiden Längsträger werden an den, jeweils an die Deformation angrenzenden Wirbelkörper fixiert. Je nach Art der Korrektur können die Längsträger vorgebogen werden. In die, von den beiden Längsträgern überbrückten Wirbelkörper, werden nun Verankerungselemente angebracht (Pedikelschrauben oder Pedikelhaken). Mit Hilfe dieser Elemente können nun die einzelnen Wirbelkörper an den Längsträger herangezogen und fixiert werden. Diese Technik kann, trotz ihrer eindeutigen Vorteile gegenüber allen bisher bekannten Methoden nur sehr selten angewendet werden, da die Verankerungselemente keine genügenden Adaptationsmöglichkeiten besitzen. Neben der gewollten axialen Zugbeanspruchung der Verankerungselemente, werden diese auch unnötigen Biegebeanspruchungen ausgesetzt, da sie rigid mit dem Längsträger verbunden werden müssen. Diese zusätzlichen Beanspruchungen sind zum Teil so gross, dass es bei älteren Fehlstellungen der Wirbelsäule, zum Ausreissen der Verankerungselemente kommen kann. Dieses Problem des Ausreissens ist sehr häufig, da die Fixation der Verankerungselemente mit dem Längsstab nur in einer Stellung erfolgen kann, d.h. wenn die Korrektur der Wirbelsäule, durch ihre "Verknöcherung" nicht 100%ig durchführbar ist, kann diese teilweise durchgeführte Korrektur nicht in dieser Stellung gehalten werden. Aus diesem Grund wird oft zuviel Kraft aufgewendet, um die Korrektur trotzdem noch 100%ig durchzuführen, und zwar nicht wegen der Korrektur selbst, sondern wegen der notwendigen Verbindung vom Verankerungselement zum Längsträger. Die einzige Alternative ist die Zwischenschaltung eines dritten Längsträgers, welcher wiederum mittels eines Verbindungselementes mit einem bestehenden Längsträger verbunden wird. Diese Menge an Implantaten liegt jedoch oft jenseits der biologischen Toleranzgrenze und beeinträchtigt die Funktion der Wirbelsäulen-Muskulatur.

Einzelne bekannte Verbindungselemente, z.B. gemäss WO92/03100 besitzen eine verbesserte Adaptation, sind jedoch vor allem bei jungen Patienten an der Toleranzgrenze, was das Volumen betrifft. Das grosse Volumen erweist sich vor allem bei komplexeren Korrekturen als negativ, da fast jeder Wirbelkörper mit einem solchen Implantat versehen wird und die Wirbelsäulen-Muskulatur stark darunter leidet.

Hier will die Erfindung Abhilfe schaffen. Der Erfindung liegt die Aufgabe zugrunde, ein Wirbelsäulenfixationselement zu schaffen, welches optimale Korrekturmöglichkeiten der Wirbelsäule erlaubt, unter gleichzeitiger Reduktion des Implantatvolumens auf ein Minimum.

Die Erfindung löst die gestellte Aufgabe mit einem Wirbelsäulenfixationselement, welches die Merkmale des Anspruchs 1 aufweist und einer Wirbelsäulenfixationseinrichtung, welche die Merkmale des Anspruchs 5 aufweist.

Die Grundidee des erfindungsgemässen Wirbelsäulenfixationselementes liegt in der Art der Kupplung zwischen den einzelnen Wirbelsäulenfixationselementen und dem Längsträger. An Stelle der bisher verwendeten, in allen Richtungen starren Verbindungen zwischen den Fixationselementen und dem Längsträger, ist die Verbindung bei der Erfindung nur in axialer Zugrichtung festgelegt. Das heisst, dass jeder einzelne Wirbelkörper durch Zug an den Längsträger herangezogen wird. Dabei spielt es keine Rolle, ob die Korrektur 100%ig durchgeführt werden kann oder nicht. Durch die in axialer Richtung stabile Fixation der Fixationselemente mit dem Längsträger, wird der Wirbelkörper in der korrigierten Stellung gehalten. Die auf den Wirbelkörper einwirkende Kraft, während der Korrektur, entspricht der Krafteinwirkung eines Seiles auf den Wirbelkörper. Das hat den zusätzlichen Vorteil, dass sich der Wirbelkörper, während des Korrekturvorganges seinen "Weg" selber suchen kann. Das hat vor allem bei der Korrektur von langzeit-deformierten Wirbelsäulen gewisse Vorteile, da sich die Wirbelkörper, durch die langzeitig angehaltene Deformation selber deformiert haben und sich ineinander verhaken können.
Axial auf die Wirbelsäule einwirkende Kräfte werden nicht durch die Wirbelsäulenfixationselemente übernommen, sondern durch die Wirbelkörper übertragen. Wenn dies nicht erwünscht ist, können sowohl die Axial-Kräfte, als auch die Torsions-Kräfte von der bereits erwähnten Rahmenkonstruktion übernommen werden. Diese Rahmenkonstruktion besteht aus den bis anhin verwendeten, in alle Richtungen starren Verankerungselementen, die mit ein bis zwei Längsträgern verbunden sind. Das Anbringen dieses Rahmens ist nicht problematisch, da nur jeweils zwei starre Fixationselemente miteinander verbunden werden. Durch Distraktion der proximalen Fixationselemente zu den distal angebrachten Fixationselementen, kann der Wirbelsäulen-Bereich innerhalb des Rahmens entlastet werden. Torsions- und Axial-Belastungen werden vom Rahmen vollständig übernommen. Durch die langstreckige Überbrückung kann eine elastische Fixation der Wirbelsäule erreicht werden, die sich heilungsfördernd auf die Wirbelsäule auswirkt. Gleichzeitig wird der Längsträger das Knochenverankerungselement Interface (Pedikelschraube oder Wirbelsäulenhaken) vor Überbeanspruchung schützen. Die erfindungsgemässen Fixationselemente werden dann innerhalb des Rahmens verwendet, um die eigentliche Korrektur durchzuführen. Die Korrektur der einzelnen Wirbelkörper kann stufenweise erfolgen. Das heisst, abwechslungsweise wird jeder Wirbelkörper dosiert an den Längsträger herangezogen. Durch dieses Vorgehen wird eine Überbeanspruchung der Implantateverankerung im Knochen vermieden.

Neben der verbesserten Funktion, zeichnet sich das erfindungsgemässe Wirbelsäulenfixationselement auch durch seine einfachste Anwendung aus.

Die erfindungsgemässen Fixationselemente besitzen entweder einen oder zwei kopfseitige Schlitze, durch die, ein von der Technik her als Kabelbinder bekanntes Verbindungselement eingeführt werden kann. Solche zugstabilen Spannvorrichtungen bestehen im wesentlichen aus einer geschlossenen Schlaufe, welche über einen Verschlussteil nur in einer Richtung bewegt, d.h. verkleinert werden kann. Beispiele für solche Spannvorrichtungen sind in den deutschen Offenlegungsschriften DE-A1 40 24 334 und DE-A1 40 21 246 im Detail beschrieben, dort allerdings zur direkten Applikation am Knochen und nicht Kupplungsglied zwischen zwei osteosynthetischen Bauelementen.

Die Kopfform der erfindungsgemässen Pedikelschrauben und Pedikelhaken kann verschiedenartig ausgebildet sein. Wenn anhand von Röntgenbildern feststeht, dass eine 100%ige Korrektur erreicht werden kann, so werden Schrauben und/oder Haken verwendet, die eine dem Längsträger entsprechende Längsnute am kopfseitigen Ende besitzen. Nach erfolgter Korrektur, können bei diesen Elementen die Spannvorrichtungen durch Metallklammern ersetzt werden. Diese Metallklammern haben den Vorteil, dass sie - falls nötig - Kräfte in allen Richtungen aufnehmen können. Dadurch kann, bei einfachen Korrekturen eventuell auf einen "Rahmen" verzichtet werden. Bei einer anderen Kopfform kann die, dem Längsträger entsprechende Nute, seitlich am Implantatekopf angebracht werden. Diese Kopfform kann dann verwendet werden, wenn der Längsträger in seiner definitiven Lage seitlich vom Verankerungselement zu liegen kommt. Wiederum kann die definitive Lage des Längsträgers und des Verankerungselementes vorgängig am Röntgenbild festgestellt werden. Eine dritte Kopfform ist so ausgebildet, dass er die minimalsten Abmessungen hat und nur den Schlitz für das Einfädeln der Spannvorrichtung besitzt. Diese Verankerungselemente kommen überall dort zur Anwendung, wo das geringe Implantatevolumen im Vordergrund steht, oder dort wo die Korrektur nicht 100%ig erfolgen kann.

Bei einer weiteren Variante besitzt das Wirbelsäulenfixationselement, insbesondere bei seiner Ausführung als Wirbelsäulenhaken einen Doppelschlitz. Die beiden kopfseitig angebrachten Schlitze haben verschiedenartige Anforderungen. Der noch vor der Hakenspitze angeordnete Schlitz dient zur Aufnahme eines "Korrekturspannelementes". Wenn dieses Korrekturspannelement axiale Kräfte auf den Haken ausübt, hat dieser die Tendenz abzukippen, was dazu führt, dass die Hakenspitzen besser in den Wirbelkörper eingreifen. Der zweite Schlitz, dient zur Aufnahme des "Fixationspannelement". Dieser Schlitz liegt ca. in der Verlängerung der Hakenbasis. Nach erfolgter Korrektur wird durch diesen Schlitz eine Spannvorrichtung gesetzt, um eine verbesserte Stabilisierung des Hakens zu erreichen.

Die Wirbelsäulenfixationselemente, sowie die Längsträger können, je nach Anwendung, aus Implantatestahl, Titan, Titanlegierung, Kunststoff oder biodegradierbaren Materialien hergestellt werden. Das gleiche gilt für die Beschaffenheit der Spannvorrichtungen. Eine bevorzugte Materialkombination besteht aus Titan für die Wirbelsäulenfixationselemente sowie die Längsträger und Kunststoff für die Spannvorrichtungen. Diese Materialzusammensetzung hat den Vorteil, dass sie sich bei den modernen Nachuntersuchungsmöglichkeiten (MRI, CT usw.) nicht negativ auswirkt.

Eine Anzahl Ausführungsbeispiele der Erfindung, welche zugleich das Funktionsprinzip erläutern, sind in der Zeichnung dargestellt und werden im folgenden näher beschrieben.
Fig. 1 zeigt einen Längsschnitt durch ein erfindungsgemässes Wirbelsäulenfixationselement in Form einer Pedikelschraube, welches mittels einer Spannvorrichtung an einen Längsträger herangezogen werden kann;
Fig. 2 zeigt einen axial um 90° verdrehten Längsschnitt durch das Wirbelsäulenfixationselement gemäss Fig. 1;
Fig. 3 zeigt einen Längsschnitt durch ein erfindungsgemässes Wirbelsäulenfixationselement mit einem Doppelschlitz;
Fig. 4 zeigt einen axial um 90° verdrehten Längsschnitt durch das Wirbelsäulenfixationselement gemäss Fig. 3;
Fig. 5 zeigt einen Längsschnitt durch ein erfindungsgemässes Wirbelsäulenfixationselement mit seitlich geneigter Längsnute im Kopfteil;
Fig. 6 zeigt einen axial um 90° verdrehten Längsschnitt durch das Wirbelsäulenfixationselement gemäss Fig. 5;
Fig. 7 zeigt einen Längsschnitt durch ein erfindungsgemässes Wirbelsäulenfixationselement mit einem miniaturisierten Kopfteil;
Fig. 8 zeigt einen axial um 90° verdrehten Längsschnitt durch das Wirbelsäulenfixationselement gemäss Fig. 7;
Fig. 9 zeigt einen Längsschnitt durch ein erfindungsgemässes Wirbelsäulenfixationselement mit einem zentralen Innensechskant im Kopfteil;
Fig. 10 zeigt einen axial um 90° verdrehten Längsschnitt durch das Wirbelsäulenfixationselement gemäss Fig. 9;
Fig. 11 zeigt einen Längsschnitt durch ein erfindungsgemässes Wirbelsäulenfixationselement in Form eines Wirbelsäulenhakens;
Fig. 12 zeigt einen Querschnitt durch einen Wirbelsäulenhaken entsprechend Fig. 11 längs der Linie A-A;
Fig. 13 zeigt einen Querschnitt durch einen Wirbelsäulenhaken entsprechend Fig. 11 längs der Linie B-B; und
Fig. 14 zeigt einen am Knochen befestigten Wirbelsäulenhaken entsprechend Fig. 11, welcher mittels einer Spannvorrichtung an einen Längsträger herangezogen werden kann.

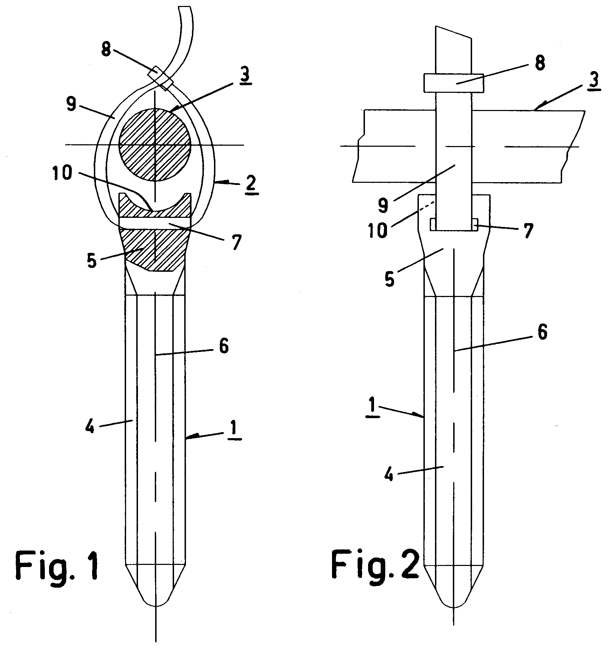

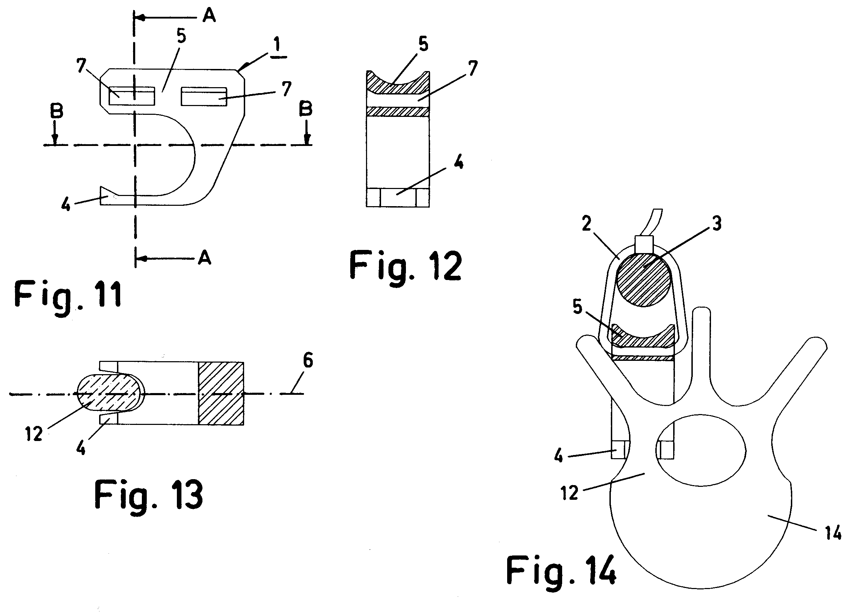

Das Wirbelsäulenfixationselement 1 ist einerseits anhand der Fig. 1 bis 10 als Pedikelschraube, anderseits anhand der Fig. 11 bis 14 als Wirbelsäulenhaken dargestellt.

Die in Fig. 1 dargestellte Wirbelsäulenfixationseinrichtung besteht aus einem erfindungsgemässen Wirbelsäulenfixationselement 1, einer zugstabilen Spannvorrichtung 2 und einem Längsträger 3. Das Wirbelsäulenfixationselement 1 besteht aus einem zur Fixation im Knochen bestimmten Verankerungsteil 4, der hier als Schraubenschaft ausgebildet ist, sowie einem daran anschliessenden, zur Befestigung an den Längsträger 3 bestimmten Kopfteil 5. Der Kopfteil weist eine quer zur Symmetrieebene 6 des Wirbelsäulenfixationselementes verlaufende, durchgängige Öffnung 7 zur Aufnahme der um den Längsträger 3 schlingbaren, zugstabilen Spannvorrichtung 2 auf.

Die zugstabile Spannvorrichtung 2 besteht ihrerseits aus einer, mittels eines Verschlussteils 8 schliessbaren und nur in einer Richtung bewegbaren, d.h. verkleinerbaren bandförmigen Schlaufe 9. Durch Festziehen der Schlaufe 2 ist es möglich das Wirbelsäulenfixationselement 1 dem Längsträger 3 zu nähern und im optimalen Fall mit diesem zur Anlage zu bringen. Zu diesem Zweck ist der Kopfteil 5 an seinem freien, zur Anlage an den Längsträger 3 bestimmten Ende mit einer konkaven Oberfläche 10 ausgebildet, welche der konvexen Oberfläche des Längsträgers 3 entspricht.

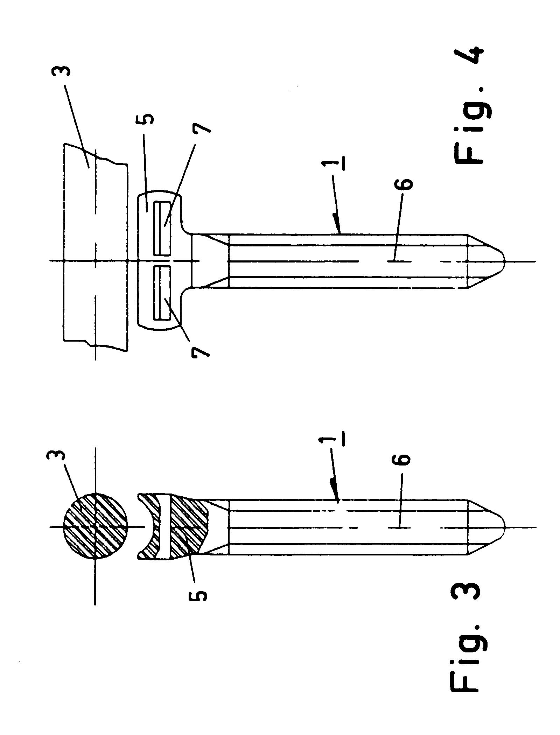

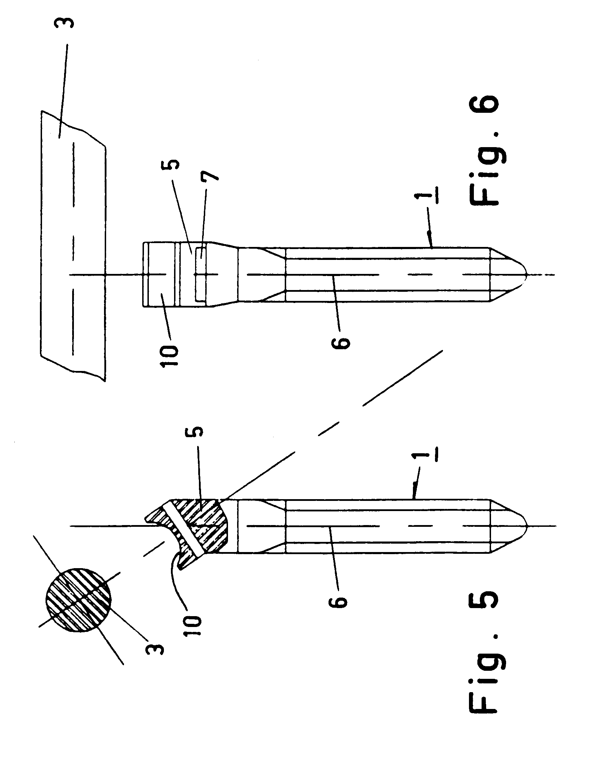

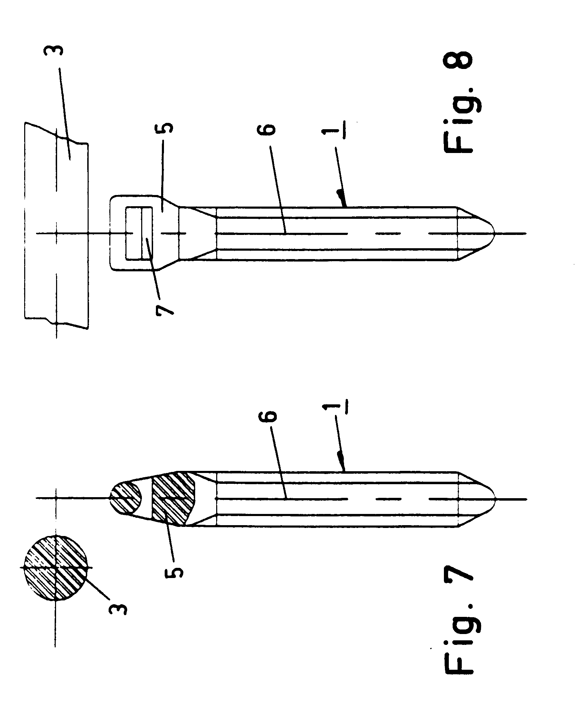

In den Fig. 3 - 8 sind verschiedene Varianten des Kopfteils 5 eines Wirbelsäulenfixationselementes 1 dargestellt.
Bei der Ausführungsform nach den Fig. 3 und 4 ist der Kopfteil 5 verbreitert um zwei separate Öffnungen 7 in Form von Schlitzen aufnehmen zu können. Bei der Variante gemäss Fig. 5 und 6 ist die konkave Oberfläche 10 des Kopfteils 5 gegenüber der Symmetrieebene 6 abgekippt um eine seitliche Anlage an den Längsträger 3 zu gestatten. Schliesslich ist bei der Variante nach den Fig. 7 und 8 der Kopfteil 5 auf ein Minimum reduziert um das Implantatvolumen möglichst klein zu halten.

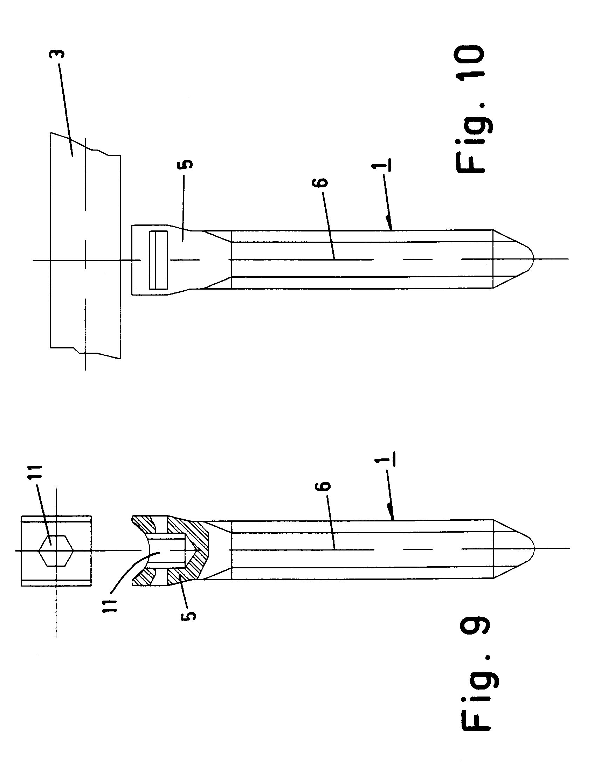

In der Fig. 9 und 10 ist ein Wirbelsäulenfixationselementes 1 dargestellt, welches in seinem Kopfteil 5 einen in Richtung der Symmetrieebene 6 verlaufenden zentralen Innensechskant 11 besitzt, der das Eindrehen der Pedikelschraube in den Knochen mit einem geeigneten Sechskant-Schraubenzieher erleichtert.

In den Figuren 11 - 14 ist ein Wirbelsäulenfixationselement 1 in Form eines Wirbelsäulenhakens dargestellt. Es besteht aus einer zur Fixation im Knochen bestimmten Verankerungsteil 4, der hier als zweischenklige Klinge ausgebildet ist, sowie einem daran anschliessenden, zur Befestigung an den Längsträger 3 bestimmten Kopfteil 5. Der Kopfteil weist zwei quer zur Symmetrieebene 13 des Wirbelsäulenfixationselementes verlaufende, durchgängige Öffnung 7 zur Aufnahme der um den Längsträger 3 schlingbaren, zugstabilen Spannvorrichtung 2 auf.
Die zweischenklige Klinge des Verankerungsteils 4 ist derart ausgebildet, dass sie den Pedikel 13 des Wirbelkörpers 14 umschliesst.

## Patentansprüche

1. Wirbelsäulenfixationselement (1) mit einem zur Fixation am Knochen bestimmten Verankerungsteil (4), insbesondere einer Pedikelschraube oder einem Pedikelhaken, sowie einer Spannvorrichtung (2), wobei der Verankerungsteil (4) einen daran anschliessenden, zur Befestigung an einen Längsträger (3) bestimmten Kopfteil (5) umfasst, welcher eine quer zur Symmetrieebene (6) des Wirbelsäulenfixationselementes (1) verlaufende, durchgängige Öffnung (7) zur Aufnahme der um den Längsträger (3) schlingbaren Spannvorrichtung (2) aufweist, **dadurch gekennzeichnet, dass** die Spannvorrichtung (2) im wesentlichen eine zugstabile, geschlossene Schlaufe (9) umfasst, welche einen Verschlussteil (8) besitzt, der nur in eine Richtung unter Verkleinerung der Schlaufe (9) bewegbar ist.

2. Wirbelsäulenfixationselement (1) nach Anspruch 1, dadurch gekennzeichnet, dass die Öffnung (7) als Schlitz zur Aufnahme einer bandförmigen Spannvorrichtung (2) ausgebildet ist.

3. Wirbelsäulenfixationselement (1) nach Anspruch 1 oder 2, dadurch gekennzeichnet, dass der Kopfteil (5) an seinem freien, zur Anlage an den Längsträger bestimmten Ende mit einer konkaven Oberfläche (10) ausgebildet ist.

4. Wirbelsäulenfixationselement (1) nach einem der Ansprüche 1 bis 3, dadurch gekennzeichnet, dass der Kopfteil (5) mehrere Öffnungen (7), vorzugsweise in Form von Schlitzen aufweist.

5. Wirbelsäulenfixationseinrichtung mit einem Wirbelsäulenfixationselement (1) nach einem der Ansprüche 1 bis 4, sowie einem Längsträger (3).

6. Wirbelsäulenfixationseinrichtung nach Anspruch 5, dadurch gekennzeichnet, dass das Wirbelsäulenfixationselement (1) und der Längsträger (3) aus Titan bestehen und die Spannvorrichtung (2) aus einem, vorzugsweise resorbierbaren, Kunststoff besteht.

7. Wirbelsäulenfixationseinrichtung nach Anspruch 5, dadurch gekennzeichnet, dass sie vollständig aus Titan oder Titanlegierungen besteht.

## Claims

1. Spinal column fixation device (1) comprising an anchoring section (4) for the purpose of fixing it to the bone, in particular a pedicle screw or pedicle hook, and further comprising a fastening device (2), the anchoring section (4) comprising an adjoining head section (5) for attachment to a longitudinal support piece (3), said head section (5) displaying an opening (7) that runs through the piece, and which runs transverse to the plane of symmetry (6) of the spinal column fixation device (1), said opening (7) being destined to receive said fastening device (2) which can be looped around the longitudinal support piece (3),
**characterized in that**
the fastening device (2) comprises essentially a tension-stable, closed loop (9) which has a closing piece (8) moveable only in one direction causing a reduction of the loop (9).

2. Spinal column fixation device (1) according to claim 1, characterized in that opening (7) is formed as a slot to admit a belt-shaped fastening device (2).

3. Spinal column fixation device (1) according to claim 1 or 2, characterized in that head section (5), on its free end meant to be in contact with the longitudinal support piece (3), is formed with a concave surface (10).

4. Spinal column fixation device (1) according to one of the claims 1 to 3, characterized in that head section (5) has several openings (7), preferably in the form of slots.

5. Spinal column fixation mechanism with a spinal column fixation device (1) according to one of claims 1 to 4, as well as a longitudinal support piece (3).

6. Spinal column fixation mechanism according to claim 5, characterized in that the spinal column fixation device (1) and the longitudinal support piece (3) consist of titanium, and the fastening device (2) consists of a preferably resorbable plastic.

7. Spinal column fixation mechanism according to claim 5, characterized in that it consists in its entirety of titanium or a titanium alloy.

## Revendications

1. Elément (1) de fixation sur la colonne vertébrale, présentant une pièce d'ancrage (4) destinée à la fixation sur l'os, en particulier une vis à pédicule ou un crochet à pédicule, ainsi qu'un dispositif de serrage (2), la pièce d'ancrage (4) comprenant une pièce de tête (5) qui s'y raccorde, qui est destinée à la fixation à un support longitudinal (3) et qui est traversée par une ouverture (7) qui s'étend transversalement par rapport au plan de symétrie (6) de l'élément (1) de fixation sur la colonne vertébrale, pour la réception du dispositif de serrage (2) qui peut être enroulé autour du support longitudinal (3), caractérisé en ce que le dispositif de serrage (2) comprend essentiellement une boucle fermée (9), résistant à la traction, qui possède une pièce de fermeture (8) qui ne peut être déplacée que dans un sens, pour raccourcir la boucle (9).

2. Elément (1) de fixation sur la colonne vertébrale selon la revendication 1, caractérisé en ce que l'ouverture (7) est configurée comme fente pour la réception d'un dispositif de serrage (2) en forme de ruban.

3. Elément (1) de fixation sur la colonne vertébrale selon la revendication 1 ou 2, caractérisé en ce qu'à son extrémité libre, destinée à venir se placer contre le support longitudinal, la pièce de tête (5) est configurée avec une surface concave (10).

4. Elément (1) de fixation sur la colonne vertébrale selon l'une quelconque des revendications 1 à 3, caractérisé en ce que la pièce de tête (5) présente plusieurs ouvertures (7), qui se présentent de préférence sous la forme de fentes.

5. Dispositif de fixation de la colonne vertébrale présentant un élément (1) de fixation sur la colonne vertébrale selon l'une quelconque des revendications 1 à 4, ainsi qu'un support longitudinal (3).

6. Dispositif de fixation de la colonne vertébrale selon la revendication 5, caractérisé en ce que l'élément (1) de fixation sur la colonne vertébrale et le support longitudinal (3) sont réalisés en titane, et en ce que le dispositif de serrage (2) est réalisé en un matériau synthétique, de préférence résorbable.

7. Dispositif de fixation de la colonne vertébrale selon la revendication 5, caractérisé en ce qu'il est entièrement réalisé en titane ou en alliages de titane.
